# EUROPEAN PATENT APPLICATION

(11) **EP 4 151 247 A1**
(43) Date of publication of application: **22.03.2023**
(21) Application number: 22818602.9
(22) Date of filing: 14.06.2022
(51) Int. Cl.: A61L 31/04, A61L 31/14, A61K 31/74, A61K 9/06, B08B 1/00, C09D 5/16, C09D 7/40

(54) **ANTI-ADHESION COMPOSITION IN FORM OF FILM WITH EXCELLENT MUCOADHESIVE AND SWELLING PROPERTIES**

(30) Priority: 14.06.2021 KR 20210076958
(71) Applicant: CNLD CO., LTD., Seoul 06373 (KR); UNIVERSITY-INDUSTRY FOUNDATION (UIF), YONSEI UNIVERSITY, Seoul 03722 (KR)
(72) Inventor: HWANG, Sung Joo, Seoul 06370 (KR); KIM, Jieun, Incheon 12915 (KR); KIM, Dongmin, Hanam-si Gyeonggi-do 21997 (KR); HWANG, Yong Kyung, Seoul 03004 (KR)
(74) Representative: Moser Götze & Partner Patentanwälte mbB
(86) International application number: PCT/KR2022/008431
(87) International publication number: WO 2022/265368

(57) **Abstract**

The present disclosure provides an anti-adhesion composition for wound protection in the form of a film comprising a biocompatible mucoadhesive polymer and a specific swellable polymer. The present disclosure relates to an anti-adhesion agent that is flexible and adheres appropriately to an application site by improving crumbling and adhesion properties, which are problems of existing film-type anti-adhesion agents.

## Description

### [Technical Field]

The present disclosure relates to an anti-adhesion composition and a method for manufacturing the same, and more particularly, to a film-form anti-adhesion composition including a muco-adhesive polymer and a swellable polymer, and a method for manufacturing the same.

### [Background Art]

Adhesion, which occurs after surgery in the process of recovery from inflammation, wounds, *etc.,* refers to the adhesion of organs and tissues around the wound site. In detail, an inflammatory reaction and a coagulation cascade occur in the wound healing process to generate fibrins. Excessive secretion of fibrins causes an increase in fibroblasts and forms new blood vessels, resulting in adhesions between tissues. The thus-formed adhesion may cause serious problems. When part of the intestine is tightened due to intra-abdominal adhesions, it may block the blood supply to the intestine. In addition, it may cause problems such as intestinal dysfunction, chronic pain, and infertility. Due to the problems described above, recently, anti-inflammatory drugs are used or medical devices for preventing adhesions by forming a physical barrier are used, and research and development relating thereto has been continued.

Previously invented anti-adhesion agents are sold in the form of a film and a liquid gel. Anti-adhesion agents in the form of liquid gel have disadvantages in that they lack mechanical durability and lack of adhesion to wound sites. However, the anti-adhesion agent in the form of a film has a characteristic in that it has an appropriate adhesion to the wound site and thus does not depart from the applied site. However, the existing film-type anti-adhesion agent lacks flexibility, and thus an experienced skill is required to apply the anti-adhesion agent to the wound site. In addition, in the case of products with a slow biodegradation rate, there are disadvantages in that the anti-adhesion agent must be removed through another surgery, *etc.*

In this regard, International Patent No. WO 2001060868A1 discloses Seprafilm^{®} (Genzyme), which is an anti-adhesion film prepared by synthesizing sodium hyaluronate and carboxymethylcellulose in a 2:1 ratio. However, Seprafilm^{®}, in which fine pores are formed during the process of removing impurities during the manufacturing process, is easily broken in the dry state and is thus not easy to handle. In addition, in the case where the surgical site is wide, it is required in a very large amount, and the film is very dry when it is brought into contact with moisture, thus requiring an advanced technique for its application. In addition, since different results occur depending on the application sites, Seprafilm^{®} has a disadvantage in that it shows limitations in use in myomectomy and laparoscopic surgeries.

In addition, International Patent No. WO 2004010854A2 discloses Surgiwrap^{®} (Mast biosurgery), which is a hydrophobic membrane in the form of an impermeable membrane with a thickness of 20 µm to 50 µm using polylactide as a biodegradable polymer peritoneal replacement film. However, since it is an impermeable membrane, the flow of body fluids can be impeded during tissue wound healing. In addition, when using Surgiwrap^{®}, it may have to be removed through revision surgery because more than 50% of the physical properties are maintained even after 6 months. In addition, it has a disadvantage in that sutures are required to fix it during surgery because it does not become gelled thus not having adhesiveness when used.

Therefore, in order to provide an ideal anti-adhesion film, it should be well attached to the wound site, and it should be flexible and easy to attach to the wound site. In addition, it should have an appropriate biodegradation rate to be decomposed in the body after a certain period of time.

### [Disclosure]

### [Technical Problem]

An object of the present disclosure is to provide an excellent anti-adhesion composition in the form of a film. Existing formulations include liquids, gels, and sols, but they lack mechanical durability as a physical barrier due to their characteristics because they have flowability. In addition, they have limited adhesion to the wound site, and thus their anti-adhesion effect is insufficient.

Existing film-type anti-adhesion agents have a disadvantage in that they cannot be freely attached to surgical sites because it is difficult for these agents to cover the surface of organs with a complex three-dimensional structure due to lack of flexibility. In addition, since the conventional film-type anti-adhesion agents are dried instantly when they are brought into contact with intraperitoneal fluid, a high level of technique is required for their attachment.

Therefore, through the present disclosure, an anti-adhesion composition can be provided in which the composition can easily be applied to a wound site and has a better anti-adhesion effect by supplementing the limitations and disadvantages of the existing film-type anti-adhesion agents.

### [Technical Solution]

The anti-adhesion composition according to the present disclosure may include a mucoadhesive polymer selected from the group including hydroxypropylmethyl cellulose, methyl cellulose, starch, polyethylene oxide, pectin, chitosan, pullulan, gelatin, carrageenan, xanthan gum, sodium carboxymethyl cellulose, sodium alginate, polyacrylic acid, and a combination thereof; a swellable polymer, which increases a barrier effect, selected from the group including calcium carboxymethylcellulose, croscarmellose sodium, crospovidone, sodium starch glycolate, low-substituted hydroxypropyl cellulose, and a combination thereof; and a plasticizer.

In one embodiment, the mucoadhesive polymer may be contained in a range of 0.1 wt% to 30 wt% based on the total weight.

In one embodiment, the swellable polymer may be contained in a range of 0.1 wt% to 30 wt% based on the total weight.

In one embodiment, the plasticizer may be selected from the group including propylene glycol, polyethylene glycol, dibutyl sebacate, diethyl phthalate, acetyl tributyl citrate, castor oil, glycerol, and a combination thereof.

In one embodiment, the plasticizer may be contained in a range of 0.1 wt% to 40 wt% based on the total weight.

In one embodiment, the anti-adhesion composition may be in the form of a film.

In one embodiment, the polyethylene glycol may be selected from the group consisting of polyethylene glycol 100, 200, 400, 1,000, 4,000, 10,000, 20,000, and a combination thereof.

In one embodiment, the anti-adhesion composition after drying may have a thickness of 0.01 mm to 2 mm.

### [Advantageous Effects]

The anti-adhesion composition according to the present disclosure can overcome the problems of the existing formulations in the form of a film. The anti-adhesion composition according to the present disclosure has appropriate flexibility when manufactured into a film, and thus it can easily be applied to parts of the body having a complex structure. In addition, the anti-adhesion composition according to the present disclosure, due to the presence of a swellable polymer, allows the film having an excellent mucoadhesion property at the application site to be instantaneously expanded when applied. Accordingly, the anti-adhesion composition according to the present disclosure can provide a more excellent anti-adhesion effect by increasing the physical barrier effect between wound sites.

The anti-adhesion composition according to the present disclosure can provide an excellent anti-adhesion effect by forming a physical barrier without departing from the wound site. The film-form anti-adhesion composition according to the present disclosure, in which a mucoadhesive polymer and a swellable polymer are mixed, is able to attach well to the mucous membrane and thereby serves as an effective physical barrier at the surgical site. More specifically, in the case of a swellable polymer, it not only attaches to the mucous membrane, but also effectively separates tissues through swelling, thereby functioning as an excellent physical barrier.

### [Description of Drawings]

FIG. 1 shows images illustrating the formulation shapes of Examples 1 to 4.
FIG. 2 shows images illustrating the surface of films, which were manufactured using a different type of swellable polymers, observed with an optical microscope.
FIG. 3 shows images illustrating the test results of anti-adhesion efficacy for formulations of Comparative Examples 1 to 3 and Example 15 through the method of Experimental Example 3.
FIG. 4 shows images illustrating the test results of anti-adhesion efficacy for formulations of Examples 9 to 16 through the method of Experimental Example 3.
FIG. 5 shows images of the formulation of Example 15 before (left) and after (right) application to the abdominal wall of a rat.

### [Mode for Disclosure]

Hereinafter, the present disclosure will be described in detail.

As used herein, the term "adhesion" may refer to a phenomenon in which skin, membranes, *etc.* that are separated from each other attach to each other. If tissue damage occurs after surgery or due to inflammation, foreign materials, bleeding, infection, wound, friction, chemical treatment, *etc.,* blood leaks out and coagulates during the healing process of the wound, resulting in an adhesion phenomenon that causes abnormal bonding between surrounding tissues. These adhesions occur frequently occur, especially after surgery. It has been known that when an adhesion to the pelvis occurs, it may become the cause of chronic pain and sexual dysfunction; an adhesion after thyroidectomy may cause side effects such as chest pain and dysphagia; an adhesion after spinal surgery may cause compression of nerves thus causing severe pain; and an adhesion within the uterus may cause infertility, amenorrhea, and habitual miscarriage.

In one embodiment, the anti-adhesion composition of the present disclosure may be prepared by dissolving a mucoadhesive polymer and a swellable polymer in a solvent to swell, and then stirring together after adding a plasticizer thereto so as to impart flexibility to the film after drying. In another embodiment, the anti-adhesion composition of the present disclosure may be prepared by adding a plasticizer to distilled water, and then adding a mucoadhesive polymer and a swellable polymer to the mixture, and then stirring them together.

Mucoadhesive polymers may be selected from the group including, for example, hydroxypropylmethyl cellulose, methyl cellulose, starch, polyethylene oxide, pectin, chitosan, pullulan, gelatin, carrageenan, xanthan gum, sodium carboxymethyl cellulose, sodium alginate, polyacrylic acid, and a combination thereof, but is not limited thereto. Since the mucoadhesive polymer can disintegrate the film when used only a small amount, it may be contained in an amount of 40 wt% to 95 wt% of the composition. Preferably, the mucoadhesive polymer may be contained in an amount of 50 wt% to 85 wt% of the composition.

The swellable polymer may be selected from the group including, for example, calcium carboxymethylcellulose, croscarmellose sodium, crospovidone, sodium starch glycolate, low-substituted hydroxypropyl cellulose, and a combination thereof, but is not limited thereto. When the swellable polymer is used in excess, it absorbs the solvent excessively, and thus other polymers cannot be dissolved and it may be precipitated after drying. Accordingly, the swellable polymer may be contained in an amount of 0.1 wt% to 30 wt% of the composition. Preferably, the swellable polymer may be contained in an amount of 5 wt% to 20 wt% of the composition.

The plasticizer may be selected from the group including, for example, propylene glycol, polyethylene glycol, dibutyl sebacate, diethyl phthalate, acetyl tributyl citrate, castor oil, glycerol, and a combination thereof, but is not limited thereto. Plasticizers are essential to improve the crumbling property, which is considered as a limitation of existing film formulations. However, when the plasticizer is used in excess, the strength of the film is lowered. Therefore, the plasticizer may be contained in an amount of 0.1 wt% to 40 wt% of the composition. Preferably, the plasticizer may be contained in an amount of 5 wt% to 20 wt% of the composition.

The solvent used to dissolve the polymer may be selected from the group including, for example, distilled water, dimethylformamide (DMF), dimethyl sulfoxide (DMSO), dimethylamine (DMA), triacetin, N-methyl-2-P Rollidone (NMP), methylpyrrolidone, alcohol, and a combination thereof, but is not limited thereto. However, since the solvent may remain in the film without being completely evaporated after drying, it is preferred that the solvent be based on distilled water, which is the safest.

A mucoadhesive polymer, a swellable polymer, a plasticizer, *etc.* are added to the solvent, and when stirring using a stirrer is completed, degassing is performed under reduced pressure. The resultant is made into a film form by pushing it to a certain thickness using a knife coating device. The resultant is placed in a hot air dryer, dried sufficiently, and then cut it into an appropriate size for use.

Due to the use of a mucoadhesive polymer and a swellable polymer, the formulation forms a physical barrier without departing from the wound site, thus rendering excellent hemostatic and anti-adhesion effects. Due to the use of a swellable polymer, the film formulation swells and exhibits mucosal adhesion when it is brought into contact with the intraperitoneal fluid; therefore, the film formulation not only instantly attaches to the wound site, but also provides a physical barrier by securing sufficient space between the wound site and the tissue, thereby exhibiting an excellent anti-adhesion effect.

### Comparative Example 1: Group with no treatment (Control)

A control group, in which no treatment was made to the site where damage was applied to the abdominal wall, was established.

### Comparative Example 2: Seprafilm^{®} (Genzyme)

A commercially available product, Seprafilm^{®} (Genzyme), was used as a film-type anti-adhesion agent consisting of hyaluronic acid and carboxymethyl cellulose.

### Comparative Example 3: Surgiwrap^{®} (Mast Biosurgery)

A commercially available product, Surgiwrap^{®} (Mast Biosurgery), was used as a film-type anti-adhesion agent consisting of poly(L-lactide-co-D-L-lactide).

### Example 1

For the anti-adhesion effect, mucoadhesive polymers (sodium alginate and chitosan) and a swellable polymer (sodium starch glycolate) were added to distilled water (100 mL) in predetermined amounts and stirred until they were evenly spread in the solvent. The stirred mixture was cast after defoaming and dried with hot air at 60°C in a drying oven.

Table 1 shows the composition of Example 1.

**[Table 1]**

| Example 1 | |
|---|---|
| Component | wt% |
| Sodium Alginate | 42.5 |
| Chitosan | 42.5 |
| Sodium Starch Glycolate | 15 |

### Example 2

For the anti-adhesion effect, a plasticizer (PEG 400) was added to distilled water (100 mL), and then mucoadhesive polymers (sodium alginate and chitosan) and a swellable polymer (sodium starch glycolate) were added to the mixture in predetermined amounts and stirred until they were evenly spread in the solvent. The stirred mixture was cast after defoaming and dried with hot air at 60°C in a drying oven.

Table 2 shows the composition of Example 2.

**[Table 2]**

| Example 2 | |
|---|---|
| Component | wt% |
| Sodium Alginate | 40 |
| Chitosan | 40 |
| Sodium Starch Glycolate | 15 |
| PEG 400 | 5 |

### Example 3

For the anti-adhesion effect, a plasticizer (PEG 400) was added to distilled water (100 mL), and then mucoadhesive polymers (sodium alginate and chitosan) and a swellable polymer (sodium starch glycolate) were added to the mixture in predetermined amounts and stirred until they were evenly spread in the solvent. The stirred mixture was cast after defoaming and dried with hot air at 60°C in a drying oven.

Table 3 shows the composition of Example 3.

**[Table 3]**

| Example 3 | |
|---|---|
| Component | wt% |
| Sodium Alginate | 42.5 |
| Chitosan | 42.5 |
| Sodium Starch Glycolate | 10 |
| PEG 400 | 5 |

### Example 4

For the anti-adhesion effect, a plasticizer (PEG 400) was added to distilled water (100 mL), and then mucoadhesive polymers (sodium alginate and chitosan) and a swellable polymer (sodium starch glycolate) were added to the mixture in predetermined amounts and stirred until they were evenly spread in the solvent. The stirred mixture was cast after defoaming and dried with hot air at 60°C in a drying oven.

Table 4 shows the composition of Example 4.

**[Table 4]**

| Example 4 | |
|---|---|
| Component | wt% |
| Sodium Alginate | 37.5 |
| Chitosan | 37.5 |
| Sodium Starch Glycolate | 20 |
| PEG 400 | 5 |

### Example 5

For the anti-adhesion effect, a plasticizer was added to distilled water (100 mL), and then mucoadhesive polymers (sodium alginate and hydroxypropylmethyl cellulose (HPMC)) and a swellable polymer (sodium starch glycolate) were added to the mixture in predetermined amounts and stirred until they were evenly spread in the solvent. The stirred mixture was cast after defoaming and dried with hot air at 60°C in a drying oven.

Table 5 shows the composition of Example 5.

**[Table 5]**

| Example 5 | |
|---|---|
| Component | wt% |
| Sodium Alginate | 40 |
| HPMC | 40 |
| Sodium Starch Glycolate | 15 |
| PEG 400 | 5 |

### Example 6

For the anti-adhesion effect, a plasticizer (PEG 400) was added to distilled water (100 mL), and then mucoadhesive polymers (sodium alginate and HPMC) and a swellable polymer (sodium croscarmellose) were added to the mixture in predetermined amounts and stirred until they were evenly spread in the solvent. The stirred mixture was cast after defoaming and dried with hot air at 60°C in a drying oven.

Table 6 shows the composition of Example 6.

**[Table 6]**

| Example 6 | |
|---|---|
| Component | wt% |
| Sodium Alginate | 40 |
| HPMC | 40 |
| Sodium Croscarmellose | 15 |
| PEG 400 | 5 |

### Example 7

For the anti-adhesion effect, a plasticizer (PEG 400) was added to distilled water (100 mL), and then mucoadhesive polymers (sodium alginate and HPMC) and a swellable polymer (crospovidone) were added to the mixture in predetermined amounts and stirred until they were evenly spread in the solvent. The stirred mixture was cast after defoaming and dried with hot air at 60°C in a drying oven.

Table 7 shows the composition of Example 7.

**[Table 7]**

| Example 7 | |
|---|---|
| Component | wt% |
| Sodium Alginate | 40 |
| HPMC | 40 |
| Crospovidone | 15 |
| PEG 400 | 5 |

### Example 8

For the anti-adhesion effect, a plasticizer (PEG 400) was added to distilled water (100 mL), and then mucoadhesive polymers (sodium alginate and HPMC) and a swellable polymer (low-substituted hydroxypropyl cellulose (L-HPC)) were added to the mixture in predetermined amounts and stirred until they were evenly spread in the solvent. The stirred mixture was cast after defoaming and dried with hot air at 60°C in a drying oven.

Table 8 shows the composition of Example 8.

**[Table 8]**

| Example 8 | |
|---|---|
| Component | wt% |
| Sodium Alginate | 40 |
| HPMC | 40 |
| L-HPC | 15 |
| PEG 400 | 5 |

### Example 9

For the anti-adhesion effect, a plasticizer (PEG 400) was added to distilled water (100 mL), and then mucoadhesive polymers (sodium alginate and pullulan) and a swellable polymer (sodium starch glycolate) were added to the mixture in predetermined amounts and stirred until they were evenly spread in the solvent. The stirred mixture was cast after defoaming and dried with hot air at 60°C in a drying oven.

Table 9 shows the composition of Example 9.

**[Table 9]**

| Example 9 | |
|---|---|
| Component | wt% |
| Sodium Alginate | 40 |
| Pullulan | 40 |
| Sodium Starch Glycolate | 15 |
| PEG 400 | 5 |

### Example 10

For the anti-adhesion effect, a plasticizer (PEG 400) was added to distilled water (100 mL), and then mucoadhesive polymers (sodium alginate and polyacrylic acid) and a swellable polymer (sodium starch glycolate) were added to the mixture in predetermined amounts and stirred until they were evenly spread in the solvent. The stirred mixture was cast after defoaming and dried with hot air at 60°C in a drying oven.

Table 10 shows the composition of Example 10.

**[Table 10]**

| Example 10 | |
|---|---|
| Component | wt% |
| Sodium Alginate | 40 |
| Polyacrylic Acid | 40 |
| Sodium Starch Glycolate | 15 |
| PEG 400 | 5 |

### Example 11

For the anti-adhesion effect, a plasticizer (PEG 400) was added to distilled water (100 mL), and then mucoadhesive polymers (sodium alginate and sodium carboxymethyl cellulose (SCMC)) and a swellable polymer (sodium starch glycolate) were added to the mixture in predetermined amounts and stirred until they were evenly spread in the solvent. The stirred mixture was cast after defoaming and dried with hot air at 60°C in a drying oven.

Table 11 shows the composition of Example 11.

**[Table 11]**

| Example 11 | |
|---|---|
| Component | wt% |
| Sodium Alginate | 40 |
| SCMC | 40 |
| Sodium Starch Glycolate | 15 |
| PEG 400 | 5 |

### Example 12

For the anti-adhesion effect, a plasticizer (PEG 400) was added to distilled water (100 mL), and then mucoadhesive polymers (sodium alginate and carrageenan) and a swellable polymer (sodium starch glycolate) were added to the mixture in predetermined amounts and stirred until they were evenly spread in the solvent. The stirred mixture was cast after defoaming and dried with hot air at 60°C in a drying oven.

Table 12 shows the composition of Example 12. Table 12 shows the composition of Example 12.

**[Table 12]**

| Example 12 | |
|---|---|
| Component | wt% |
| Sodium Alginate | 40 |
| Carrageenan | 40 |
| Sodium Starch Glycolate | 15 |
| PEG 400 | 5 |

### Example 13

For the anti-adhesion effect, a plasticizer (PEG 400) was added to distilled water (100 mL), and then mucoadhesive polymers (sodium alginate and pectin) and a swellable polymer (sodium starch glycolate) were added to the mixture in predetermined amounts and stirred until they were evenly spread in the solvent. The stirred mixture was cast after defoaming and dried with hot air at 60°C in a drying oven.

Table 13 shows the composition of Example 13.

**[Table 13]**

| Example 13 | |
|---|---|
| Component | wt% |
| Sodium Alginate | 40 |
| Pectin | 40 |
| Sodium Starch Glycolate | 15 |
| PEG 400 | 5 |

### Example 14

For the anti-adhesion effect, a plasticizer (PEG 400) was added to distilled water (100 mL), and then mucoadhesive polymers (sodium alginate and pectin) and a swellable polymer (sodium starch glycolate) were added to the mixture in predetermined amounts and stirred until they were evenly spread in the solvent. The stirred mixture was cast after defoaming and dried with hot air at 60°C in a drying oven.

Table 14 shows the composition of Example 14.

**[Table 14]**

| Example 14 | |
|---|---|
| Component | wt% |
| Sodium Alginate | 30 |
| Pectin | 50 |
| Sodium Starch Glycolate | 15 |
| PEG 400 | 5 |

### Example 15

For the anti-adhesion effect, a plasticizer (PEG 400) was added to distilled water (100 mL), and then mucoadhesive polymers (sodium alginate and pectin) and a swellable polymer (sodium starch glycolate) were added to the mixture in predetermined amounts and stirred until they were evenly spread in the solvent. The stirred mixture was cast after defoaming and dried with hot air at 60°C in a drying oven.

Table 15 shows the composition of Example 15.

**[Table 15]**

| Example 15 | |
|---|---|
| Component | wt% |
| Sodium Alginate | 50 |
| Pectin | 30 |
| Sodium Starch Glycolate | 15 |
| PEG 400 | 5 |

### Example 16

For the anti-adhesion effect, a plasticizer (PEG 400) was added to distilled water (100 mL), and then mucoadhesive polymers (sodium alginate and pectin) and a swellable polymer (sodium starch glycolate) were added to the mixture in predetermined amounts and stirred until they were evenly spread in the solvent. The stirred mixture was cast after defoaming and dried with hot air at 60°C in a drying oven.

Table 16 shows the composition of Example 16.

**[Table 16]**

| Example 16 | |
|---|---|
| Component | wt% |
| Sodium Alginate | 60 |
| Pectin | 20 |
| Sodium Starch Glycolate | 15 |
| PEG 400 | 5 |

### Experimental Example 1: Evaluation of thickness uniformity

After mixing all of the compositions and drying the films cast with a knife coating device (KP-3000V), the uniformity of film thickness was evaluated. The uniformity evaluation was measured three times for each film (Table 17).

In the film of Example 1 in which no plasticizer was added, precipitation of polymers was observed. In addition, as the polymers were precipitated, the thickness of the formulation was non-uniform. In contrast, in the film of Example 2 in which a plasticizer was added, polymer precipitation was not observed any more, and the non-uniformity in thickness of the formulation was also resolved. In the films of Examples 3 and 4 prepared by varying the content of the swellable polymer, the film prepared was not flat or polymers were precipitated.

**[Table 17]**

| Example | Thickness (mm) | Relative Standard Deviation (%) |
|---|---|---|
| 1 | 0.82±0.3 | 36.6 |
| 2 | 0.62±0.05 | 8.1 |
| 3 | 0.74±0.3 | 40.5 |
| 4 | 0.83±0.4 | 48.2 |

### Experimental Example 2: Evaluation of surface uniformity

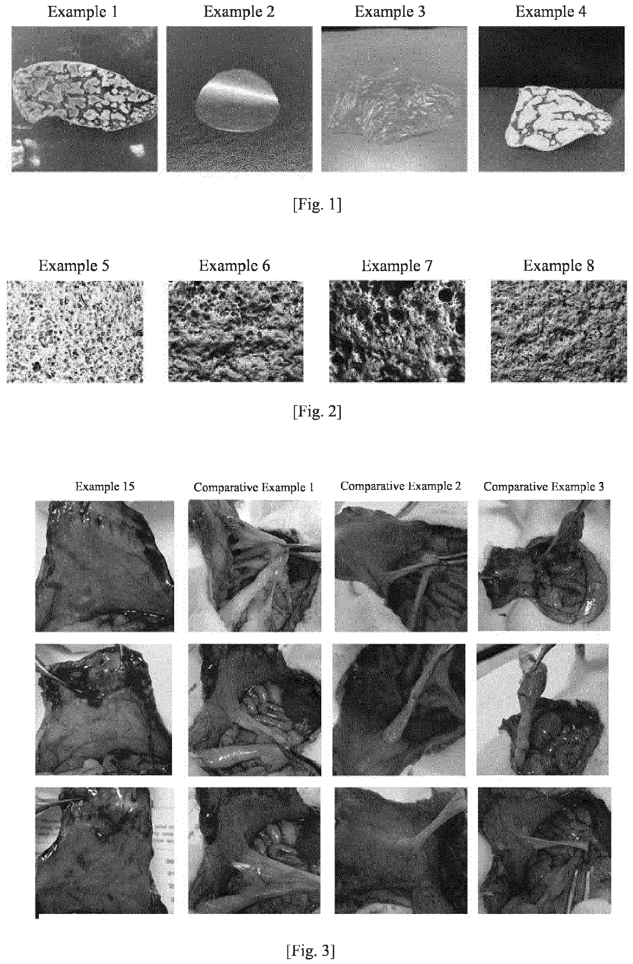

In order to select a suitable swellable polymer, different types of swellable polymers were tested and the structure of each film was examined under an optical microscope. As a result of observing the films of Examples 5 to 8, as shown in FIG. 2, the film of Example 5 had the most uniform and smoothest surface.

### Experimental Example 3: Animal experiment for anti-adhesion effect of anti-adhesion agent

Sprague-Dawley rats were anesthetized with isoflurane using a small anesthesia device, and an incision was made in the center of the abdomen. Then, in order to induce the formation of adhesions, the left abdominal wall of the open abdominal cavity was abrased 200 times using a 1 cm × 1 cm piece of sandpaper. Each sample of Comparative Examples and Examples was applied to the damaged abdominal wall. After 10 days of the application, laparotomy was performed and the adhesions were evaluated by visual observation whether adhesions were formed on the abrased abdominal wall.

The evaluation of adhesions by visual observation was conducted by Ntourakis, Dimitrios *et al.* (Ntourakis, Dimitrios, Michail Katsimpoulas, Anna Tanoglidi, Calypso Barbatis, Panayotis E Karayannacos, Theodoros N Sergentanis, Nikolaos Kostomitsopoulos, and Anastasios Machairas. "Adhesions and Healing of Intestinal Anastomoses: The Effect of Anti-Adhesion Barriers." Surgical Innovation 23, No. 3 (2016): 266-76). Specifically, the degree of adhesion was evaluated by classification (0 to 4, Table 18) according to the size, thickness, and force required to separate the adhesions on the adhesion surface, and the results are shown in Table 19.

**[Table 18]**

| Score | Description |
|---|---|
| 0 | formation of no adhesion |
| 1 | formation of thin, film-like adhesion and easy separation without much strength |
| 2 | thick and a little strength is required to separate the adhesion to be pulled taut |
| 3 | thick and it is impossible to separate adhesion without scissors (i.e., it is impossible to separate adhesion by the pulling force alone) |
| 4 | formation of adhesion by intermingling with organs in the abdominal cavity |

After performing an experiment by the method described in Experimental Example 3, the degree of adhesion was evaluated according to the score recording method shown in Table 18.

**[Table 19]**

| Test Group | No. 1 | No. 2 | No. 3 | Mean Score of Adhesion Evaluation (n = 3) |
|---|---|---|---|---|
| Comparative Example 1 | 3 | 4 | 4 | 3.67 |
| Comparative Example 2 | 2 | 2 | 2 | 2 |

| Comparative Example 3 | 1 | 0 | 2 | 1 |
|---|---|---|---|---|
| Example 9 | 1 | 2 | 1 | 1.33 |
| Example 10 | 2 | 2 | 2 | 2 |
| Example 11 | 0 | 1 | 1 | 0.67 |
| Example 12 | 1 | 0 | 1 | 0.67 |
| Example 13 | 0 | 0 | 0 | 0 |
| Example 14 | 1 | 1 | 1 | 1 |
| Example 15 | 0 | 0 | 0 | 0 |
| Example 16 | 0 | 1 | 1 | 0.67 |

As shown in FIG. 3, it was confirmed that the film of Example 15 of the present disclosure had a more effective anti-adhesion effect in the degree of adhesion, adhesion strength, adhesion area, *etc.* compared to those of the untreated group (Comparative Example 1) or commercially available Seprafilm^{®} (Genzyme) (Comparative Example 2) and Surgiwrap^{®} (Mast Biosurgery) (Comparative Example 3).

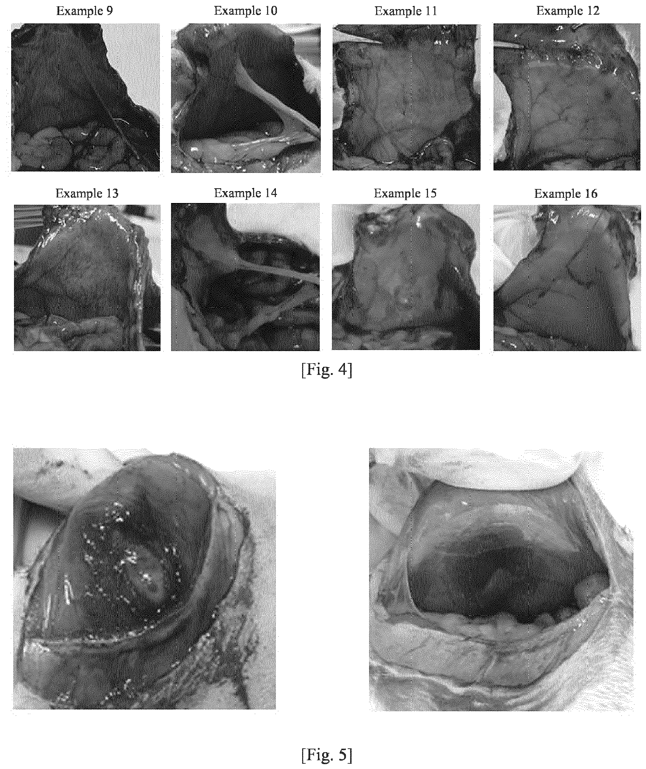

In addition, the test results of the anti-adhesion effect of the films of Examples 9 to 16 of the present disclosure through the method of Experimental Example 3 are shown in FIG. 4. As shown in FIG. 4, it can be confirmed that the anti-adhesion composition according to the present disclosure has an extremely excellent anti-adhesion effect.

## Claims

1. An anti-adhesion composition comprising:
a mucoadhesive polymer selected from the group including hydroxypropylmethyl cellulose, methyl cellulose, starch, polyethylene oxide, pectin, chitosan, pullulan, gelatin, carrageenan, xanthan gum, sodium carboxymethyl cellulose, sodium alginate, polyacrylic acid, and a combination thereof;
a swellable polymer, which increases a barrier effect, selected from the group including calcium carboxymethylcellulose, croscarmellose sodium, crospovidone, sodium starch glycolate, low-substituted hydroxypropyl cellulose, and a combination thereof; and
a plasticizer.

2. The anti-adhesion composition of claim 1, wherein the mucoadhesive polymer is contained in a range of 0. 1 wt% to 30 wt% based on the total weight.

3. The anti-adhesion composition of claim 1, wherein the swellable polymer is contained in a range of 0. 1 wt% to 30 wt% based on the total weight.

4. The anti-adhesion composition of claim 1, wherein the plasticizer is selected from the group including propylene glycol, polyethylene glycol, dibutyl sebacate, diethyl phthalate, acetyl tributyl citrate, castor oil, glycerol, and a combination thereof.

5. The anti-adhesion composition of claim 1, wherein the plasticizer is contained in a range of 0. 1 wt% to 40 wt% based on the total weight.

6. The anti-adhesion composition of claim 1, wherein the anti-adhesion composition is in the form of a film.

7. The anti-adhesion composition of claim 4, wherein the polyethylene glycol is selected from the group consisting of polyethylene glycol 100, 200, 400, 1,000, 4,000, 10,000, 20,000, and a combination thereof.

8. The anti-adhesion composition of claim 6, wherein the anti-adhesion composition after drying has a thickness of 0.01 mm to 2 mm.
